# EUROPEAN PATENT APPLICATION

(11) **EP 4 414 455 A1**
(43) Date of publication of application: **14.08.2024**
(21) Application number: 22878559.8
(22) Date of filing: 05.10.2022
(51) Int. Cl.: C12N 15/62, C12N 5/10, C12N 15/11, C12N 15/85

(54) **STRUCTURE**

(30) Priority: 07.10.2021 JP 2021165716
(71) Applicant: H.U. Group Research Institute G.K., Akiruno-shi, Tokyo 197-0833 (JP)
(72) Inventor: KAWASAKI, Yuki, Akiruno-shi, Tokyo 197-0833 (JP); TANAKA, Kosei, Akiruno-shi, Tokyo 197-0833 (JP); OKA, Yuma, Akiruno-shi, Tokyo 197-0833 (JP)
(74) Representative: Müller-Boré & Partner Patentanwälte PartG mbB
(86) International application number: PCT/JP2022/037314
(87) International publication number: WO 2023/058691

(57) **Abstract**

The present invention provides a means useful for delivering a large amount of a functional molecule. More specifically, the present invention provides a construct containing an extracellular vesicle (EV) loading signal-containing nucleic acid and a functional molecule linked thereto,
wherein the EV loading signal-containing nucleic acid contains an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 genes, and
wherein the functional molecule is a functional molecule other than an EV loading signal-containing nucleic acid.

## Description

### Field

The present invention relates to a construct containing an extracellular vesicle loading signal-containing nucleic acid and a functional molecule linked thereto.

### Background

Extracellular vesicles (EVs), which are small vesicles secreted from various types of cells and having a membrane structure, are present in body fluids such as blood or a cell culture medium. EVs contain various substances bearing functions such as intercellular information transmission. Because of this, EVs have been analyzed for purposes such as diagnosis and drug discovery.

Use of EVs as vehicles for delivering substances has been studied. For example, Patent Literature 1 discloses a method of encapsulating a polypeptide in EVs. Furthermore, Patent Literature 2 discloses a method of encapsulating a medical drug in EVs by using a metabolic pathway of a cell cultured in the presence of a medical drug linked to a metabolic component (e.g., lipids, peptides).

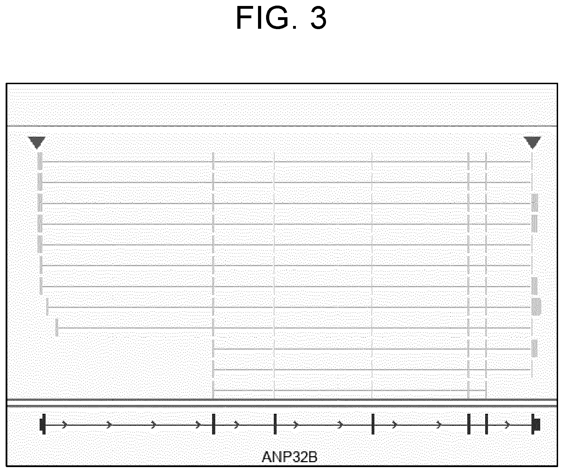

Meanwhile, Non-Patent Literature 1 teaches that various types of RNAs are loaded into EVs secreted from colorectal cancer cells. For example, it teaches that, with respect to non-coding RNAs, CDT-2328D6.1 gene is most selectively loaded into both colorectal cancer KRAS mutant cell line (DKO-1)-derived EVs and KRAS wild-type cell line (DKs-8)-derived EVs, and that, with respect to coding RNAs, HHLA1 gene is most selectively loaded into DKO-1-derived EVs, whereas CYSLTR1 gene is most selectively loaded into in DKs-8-derived EVs (Table 1). It also teaches that an RNA product of RAB13 gene was found in the DKO-1-derived EVs but not in DKs-8-derived EVs (e.g., FIGs. 3B, 3C).

### Citation List

### Patent Literature

Patent Literature 1: WO 2017/203260 A
Patent Literature 2: WO 2018/011128 A

### Non Patent Literature

Non Patent Literature 1: Hinger et al., Cell Rep. 2018 Oct 16; 25(3): 715-725.e4

### Summary

### Technical Problem

It is an object of the present invention to provide a means useful for delivering a large amount of a functional molecule.

### Solution to Problem

As a result of intensive studies, the present inventors have found that a construct containing an EV loading signal-containing nucleic acid and a functional molecule thereto can be used for delivering a large amount of the functional molecule. Culturing an animal cell, which was obtained by introducing such a nucleic acid construct therein, in a culture medium, allows a functional molecule to be selectively loaded into EVs taking an advantage of the ability of an EV loading signal-containing nucleic acid to be loaded into EVs. The present inventors have also succeeded in identifying EV loading signal-containing nucleic acids having excellent abilities to be loaded into EVs that can be used in such a construct, and thus have achieved the present invention.

More specifically, the present invention is as follows.
[1] A construct comprising: an extracellular vesicle (EV) loading signal-containing nucleic acid; and a functional molecule linked thereto,
   wherein the EV loading signal-containing nucleic acid contains an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 genes and
   wherein the functional molecule is a functional molecule other than the EV loading signal-containing nucleic acid.
[2] The construct according to [1], wherein the EV loading signal-containing nucleic acid contains an EV loading signal in one or more genes selected from the group consisting of RAB13, LUC7L3, ANP32B, NET1, MTURN, C22orf46, PLEKHA4, HMGN5, FBXW4, ZSWIM9, CA11 and LINC01952 genes.
[3] The construct according to [1] or [2], wherein the EV loading signal-containing nucleic acid contains a 3'-untranslated region in the one or more genes or a partial region thereof having an ability to be loaded into EVs.
[4] The construct according to any of [1] to [3], wherein the EV loading signal-containing nucleic acid is a nucleic acid containing (i) a nucleotide sequence consisting of nucleotide residues of positions 783 to 1164 in a nucleotide sequence of SEQ ID NO: 1 or (ii) a nucleotide sequence having an identity of 90% or more to a nucleotide sequence consisting of nucleotide residues of positions 783-1037 in a nucleotide sequence of SEQ ID NO: 1, and having an ability to be loaded into EVs.
[5] The construct according to [1], wherein the EV loading signal-containing nucleic acid is RNA.
[6] The construct according to any of [1] to [5], wherein the functional molecule is a medicine or a diagnostic reagent.
[7] The construct according to any of [1] to [6], wherein the functional molecule is a nucleic acid molecule, an amino acid, a peptide, a protein, a lipid, a monosaccharide or a polysaccharide.
[8] The construct according to any of [1] to [7], wherein the construct is a nucleic acid construct containing an EV loading signal-containing nucleic acid and a functional nucleic acid molecule linked thereto.
[9] The construct according to [8], wherein the nucleic acid construct is the following (1) or (2):
   (1) a DNA construct containing an EV loading signal-containing DNA and a DNA linked thereto; or
   (2) an RNA construct containing an EV loading signal-containing RNA and an RNA linked thereto.
[10] An expression vector comprising: the nucleic acid construct according to [8] or [9]; and a promoter operably linked thereto.
[11] A transformed cell comprising an expression unit containing the nucleic acid construct according to [8] or [9]; and a promoter operably linked thereto.
[12] The transformed cell according to [11], wherein the transformed cell is a kidney cell, a breast adenocarcinoma cell or a non-small cell lung cancer cell.
[13] The transformed cell according to [11] or [12], wherein the transformed cell is a HEK293 cell line, an NCI-H1299 cell line or an MCF7 cell line.
[14] An RNA transcript of a nucleic acid construct comprising: an EV loading signal-containing nucleic acid; and a functional nucleic acid molecule transcribably linked thereto,
   wherein the EV loading signal-containing nucleic acid contains an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 genes, and
   wherein the functional molecule is a functional nucleic acid molecule other than the EV loading signal-containing nucleic acid.
[15] An EV comprising the RNA transcript according to [14].
[16] The EV according to [15], wherein the EV is a CD9-positive extracellular vesicle.
[17] A method for producing an EV containing a functional molecule, comprising the following (1) and (2):
   (1) introducing the construct according to any of [1] to [9] or the expression vector according to [10] into an animal cell; and
   (2) culturing the animal cell obtained in (1) in a culture medium to secrete the EV containing a functional molecule from the animal cell into the culture medium.
[18] The method according to [17], wherein the animal cell is a kidney cell, a breast adenocarcinoma cell or a non-small cell lung cancer cell line.
[19] The method according to [17] or [18], wherein the animal cell is a HEK293 cell line, an NCI-H1299 cell line or an MCF7 cell line.
[20] A method for producing an EV containing an RNA transcript of a nucleic acid construct containing an EV loading signal-containing nucleic acid and a functional nucleic acid molecule linked thereto, the method comprising culturing the transformed cell according to any of [11] to [13] to produce the EV containing the RNA transcript.

### Effects of Invention

According to the invention, it is possible for any functional molecule to be selectively loaded into EVs. Thus, according to the present invention, EVs can be used as a means for delivering a large amount of a functional molecule.

### Brief Description of Drawings

FIG. 1 is a diagram illustrating mRNA of RAB13 gene detected in extracellular vesicles. Upper stage: mRNA detected; lower stage: reference transcript of the NCBI RefSeq database used in IGV_2.8.12 (the same applies to FIGs. 2 and 3).
FIG. 2 is a diagram illustrating mRNA of NET1 gene detected from EVs.
FIG. 3 is a diagram illustrating mRNA of ANP32B gene detected from EVs.
FIG. 4 is a graph illustrating the EV loading rates of foreign genes in a transient expression experiment.
FIG. 5 is a graph illustrating the EV loading rates of foreign genes in a stably expressing cell line.
FIG. 6 is a graph illustrating the EV loading rates of foreign genes using a partial sequence of RAB13 gene.
FIG. 7 is a graph illustrating loading to EVs using cells other than the HEK293 cell line.
FIG. 8 is a graph illustrating loading to EVs using an eGFP fusion gene.

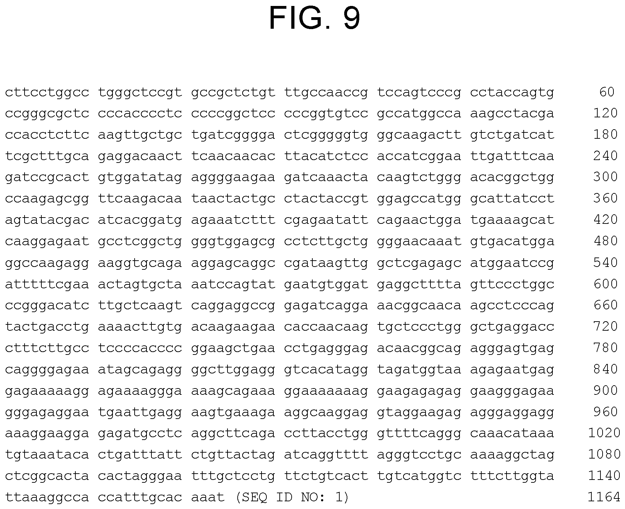

FIG. 9 illustrates a full-length DNA sequence (SEQ ID NO: 1) of RAB13 gene.
FIG. 10 is a graph illustrating the EV loading rates of foreign genes using a RAB13 3' UTR partial sequence. S: sense strand, AS: antisense strand

### DESCRIPTION OF EMBODIMENTS

The present invention provides a construct containing an extracellular vesicle (EV) loading signal-containing nucleic acid and a functional molecule thereto. The EV loading signal-containing nucleic acid is a nucleic acid containing an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 genes.

The number of genes to be selected for the EV loading signal is not particularly limited as long as it is one or more, but may be preferably one to five, more preferably one to three, still further preferably one or two, and particularly preferably one.

The EV loading signal-containing nucleic acid may contain a single EV loading signal or a plurality of EV loading signals. When the EV loading signal-containing nucleic acid contains a plurality of EV loading signals, the plural EV loading signals may include EV loading signals derived from the same gene or different genes. The number of EV loading signals to be contained in an EV loading signal-containing nucleic acid may be one or more, preferably one to five, and more preferably one to three, still further preferably one or two, and particularly preferably one.

The EV loading signal-containing nucleic acid may contain an EV loading signal in the downstream or upstream region of a coding region or between the coding regions in the gene.

In a certain embodiment, the EV loading signal-containing nucleic acid may contain an EV loading signal in a gene having an ability to be more selectively loaded into EVs, among the aforementioned genes. Accordingly, as a nucleic acid containing such an EV loading signal, it is possible to use a nucleic acid containing an EV loading signal in one or more genes selected from the group consisting of RAB13, LUC7L3, ANP32B, NET1, MTURN, C22orf46, PLEKHA4, HMGN5, FBXW4, ZSWIM9, CA11 and LINC01952 genes. Of these genes, RAB13, LUC7L3, NET1, C22orf46, ZSWIM9 and LINC01952 genes having more excellent ability to be loaded into EVs are more preferable.

The EV loading signal-containing nucleic acid to be used in the present invention is not particularly limited as long as it contains an EV loading signal of a gene as mentioned above. As the EV loading signal-containing nucleic acid, for example, a nucleic acid encoding the full-length region of a gene as mentioned above may be used. Alternatively, as the EV loading signal-containing nucleic acid, a nucleic acid encoding a partial region of a gene as mentioned above having an ability to be loaded into EVs may be used. Such a partial region can be determined by preparing deletion mutants of a gene as mentioned above and evaluating their abilities to be loaded into EVs.

In a certain embodiment, the EV loading signal-containing nucleic acid may correspond to an untranslated region (e.g., 5'-UTR, 3'-UTR) in a gene as mentioned above or a partial region thereof having an ability to be loaded into EVs. Since an EV loading signal can be present within an untranslated region of a gene as mentioned above, the untranslated region or a partial region thereof can be used as the EV loading signal. A partial region of the untranslated region having an ability to be loaded into EVs can be determined by preparing deletion mutants of the untranslated region and evaluating their abilities to be loaded into EVs.

In another certain embodiment, the EV loading signal-containing nucleic acid may be a nucleic acid containing (i) the nucleotide sequence consisting of nucleotide residues of positions 783 to 1164 in a nucleotide sequence of SEQ ID NO: 1 or (ii) a nucleotide sequence having an identity of 90% or more to the nucleotide sequence consisting of nucleotide residues of positions 783-1037 and having an ability to be loaded into EVs. Alternatively, the nucleotide sequence consisting of nucleotide residues of positions 783 to 1164 of the nucleotide sequence of SEQ ID NO: 1 may be partially deleted. The nucleic acid having an ability to be loaded into EVs may be DNA to be transcribed into RNA having an ability to be loaded into EVs or RNA having an ability to be loaded into EVs.

The identity of a nucleotide sequence may be 910 or more, 92% or more, 93% or more, 94% or more, 95% or more, 96% or more, 97% or more, 98% or more or, 99% or more. The identity of a nucleotide sequence can be calculated based the algorithm blastn. More specifically, the identity of a nucleotide sequence can be calculated based on the algorithm blastn (provided by NCBI) using Scoring Parameters (Match/Mismatch Scores = 1, -2; Gap Costs = Linear) set by default.

A nucleotide sequence having a desired identity to a target nucleotide sequence can be specified based on a desired number of nucleotide-residue mutations to the target nucleotide sequence. The nucleotide-residue mutations refer to one, two, three or four types of mutations selected from the group consisting of deletion, substitution, addition and insertion of nucleotide residues. The nucleotide-residue mutations may be introduced into a single region or different regions of a nucleotide sequence. For example, when a target nucleotide sequence consists of 382 nucleotide residues (nucleotide residues of positions 783 to 1164 of the nucleotide sequence of SEQ ID NO: 1), a nucleotide sequence having an identity of 90% or more to the target nucleotide sequence may have 38 or less nucleotide-residue mutations to the target nucleotide sequence. A nucleotide sequence having an identity of 95% or more to a target nucleotide sequence may have 19 or less nucleotide-residue mutations to the target nucleotide sequence. Nucleotide sequences having an identity of 96% or more, 97% or more, 98% or more and 99% or more to a target nucleotide sequence may have 15 or less, 11 or less, seven or less and three or less nucleotide-residue mutations, respectively, to the target nucleotide sequence.

The EV loading signal-containing nucleic acid may be RNA or DNA. If the functional molecule is a functional molecule (e.g., medical drug, diagnostic agent) other than a functional nucleic acid molecule, the EV loading signal-containing nucleic acid is preferably RNA. In contrast, when the functional molecule is a functional nucleic acid molecule transcribable together with an EV loading signal-containing nucleic acid, the EV loading signal-containing nucleic acid is preferably DNA or RNA.

As the functional molecule, any functional molecule can be used. For example, the functional molecule may be a medical drug or a labeling agent. Examples of the medical drug include low-molecular weight compounds and high-molecular weight compounds (e.g., protein preparations such as antibodies, polysaccharides). Furthermore, the medical drug may be a therapeutic drug for diseases such as various types of cancers, autoimmune diseases/inflammatory diseases (e.g., allergic diseases, rheumatoid arthritis, systemic lupus erythematosus), cerebral nerve diseases (e.g., cerebral infarction, Alzheimer's disease, Parkinson's disease), infections (e.g., bacterial infections, viral infections), hereditary/rare diseases and metabolic disorders (e.g., diabetes, hyperlipidemia). More specifically, examples of the medical drug include anticancer agents, cytostatic agents, tyrosine kinase inhibitors, statins, NSAIDs, antibiotics, antifungal agents, antibacterial agents, anti-inflammatory agents, antifibrotic agents, antihypertensive agents, aromatase inhibitors, esterase inhibitors, anticholinergic agents, SSRIs, BKT inhibitors, HER inhibitors, AKT inhibitors, BCR-ABL inhibitors, angiogenesis inhibitors, ALK inhibitors, BRAF inhibitors, MEK inhibitors, PI3K inhibitors, neprilysin inhibitors, BACE inhibitors, MAPK inhibitors, LSD1 inhibitors, lactamase inhibitors, IDO inhibitors, ERK inhibitors, and Chk1 inhibitors. The labelling agent is a substance that enables detection of a target (e.g., tissue, cell, substance). Examples of the labeling agent include enzymes (e.g., peroxidase, alkaline phosphatase, luciferase, β-galactosidase), affinity substances (e.g., streptavidin, biotin, digoxigenin, aptamer), fluorescent substances (e.g., fluorescein, fluorescein isothiocyanate, rhodamine, green fluorescent protein, red fluorescent protein), luminescent substances (e.g., luciferin, aequorin, acridinium ester, tris(2,2'-bipyridyl)ruthenium, luminol), and radioactive isotopes. The labeling agent may be an affinity substance (e.g., antibody, aptamer, lectin, complementary nucleic acid) labeled with a labeling agent.

The functional molecule may be a substance related to a biogenic substance. Examples of the biogenic substance include nucleic acid molecules (e.g., nucleosides, nucleotides, oligonucleotides, polynucleotides), amino acids (e.g., L-amino acids or D-amino acids and racemates of these), peptides (e.g., oligopeptides such as dipeptides and tripeptides), proteins (including e.g., modified proteins such as glycoproteins), lipids, monosaccharides and polysaccharides. These substances may be naturally occurring substances or artificially modified substances.

In a certain embodiment, the functional molecule may be a functional nucleic acid molecule. The functional nucleic acid molecule may be a nucleic acid encoding a protein (including a peptide such as an oligopeptide and a polypeptide) or a nucleic acid not encoding a protein. If the functional nucleic acid molecule is a nucleic acid encoding a protein, the functional nucleic acid molecule can be translated into a protein in a cell to which the molecule is to be delivered by EVs. Examples of the protein include labeled proteins (e.g., fluorescent proteins), immunoglobulins (e.g., antibodies), receptors (e.g., cell-membrane receptors, nuclear receptors), growth factors, enzymes, channels, ligands, adapters, cell adhesion factors, transporters, cytoplasmic proteins and transcription factors. If the functional nucleic acid molecule is a nucleic acid not encoding a protein, the functional nucleic acid molecule is a nucleic acid exerting a physiological function by itself. Examples of the functional nucleic acid molecule include antisense nucleic acids, RNA interference-inducing nucleic acids (e.g., loop-forming siRNAs capable of forming an intrachain double strand since it contains mutually complementary two nucleic acid regions within a single-stranded nucleic acid) or precursors thereof, LincRNAs, snRNAs, snoRNAs, splice switching RNAs, aptamers and ribozymes. The functional nucleic acid molecule may have a naturally occurring strand or an artificially modified strand. The functional nucleic acid molecule preferably has a naturally occurring strand if it is desired to be transcribed. In contrast, the functional nucleic acid molecule may have a naturally occurring strand if it is not desired to be transcribed or may have an artificially modified strand in order to improve a function or stability thereof. Examples of the modification include 2'-O-Me, 2'-O-MOE, 2'-F, 2'-O-allyl, LNA, CLNA, ENA, PNA, phosphorothioate and tricyclo-DNA.

The size of the functional nucleic acid molecule is not particularly limited as long as the functional nucleic acid molecule, if linked to an EV loading signal-containing nucleic acid, can realize loading of the construct of the present invention, i.e., an RNA transcript, into EVs. For example, the functional nucleic acid molecule may be constituted of 15 or more nucleotide residues. The functional nucleic acid molecule may be composed of preferably 18 or more, more preferably 20 or more, still further preferably 30 or more, particularly preferably 50 or more, 100 or more, 200 or more, 300 or more, 400 or more or 500 or more nucleotide residues. The functional nucleic acid molecule may be constituted of preferably 50,000 or less, more preferably 30,000 or less, still further preferably 20,000 or less, particularly preferably 10,000 or less, 5,000 or less, 3,000 or less or 2,000 or less nucleotide residues.

In a certain embodiment, the construct may be a nucleic acid construct containing an EV loading signal-containing nucleic acid and a functional nucleic acid molecule linked thereto. The EV loading signal-containing nucleic acid and functional nucleic acid molecule may be linked such that they can be transcribed as a unit. The EV loading signal-containing nucleic acid and functional nucleic acid molecule may be linked directly or via a linker nucleic acid. The EV loading signal-containing nucleic acid can be arranged at the 5' (upstream) or 3' (downstream) side of a functional nucleic acid molecule. Preferably, the EV loading signal-containing nucleic acid can be arranged at the 5' (upstream) or 3' (downstream) side of a functional nucleic acid molecule.

The nucleic acid construct can be used in the form of a single strand or a double strand. The nucleic acid construct can also be used as a DNA construct or RNA construct in order to realize transcription within a cell and the subsequent loading into EVs. Accordingly, the EV loading signal-containing nucleic acid to be contained in the nucleic acid construct may be DNA or RNA. The functional nucleic acid molecule is preferably DNA or RNA. If the EV loading signal-containing nucleic acid is DNA, the functional nucleic acid molecule is preferably DNA. If the EV loading signal-containing nucleic acid is RNA, the functional nucleic acid molecule is preferably RNA. This enables transcription of a nucleic acid construct within a cell and allows an RNA transcript of the nucleic acid construct to selectively be loaded into EVs.

In an embodiment, the nucleic acid construct may be a DNA construct containing an EV loading-signal containing DNA and a DNA linked thereto. The DNA construct can be used preferably in the form of a double-stranded DNA. More specifically, the DNA construct is integrated in an expression vector (e.g., plasmid, DNA vector such as viral vector) in the form of double-stranded DNA, and then, transcribed within a cell. This allows the RNA transcript of the DNA construct to be selectively loaded into EVs. Preferably, the DNA construct may be a construct that contains a nucleotide sequence having an identity of 90% or more to the nucleotide sequence consisting of nucleotide residues of positions 783 to 1164 in the nucleotide sequence of SEQ ID NO: 1 as an EV loading-signal containing DNA, and that is to be transcribed into RNA having an ability to be loaded into EVs.

In another embodiment, the nucleic acid construct may be an RNA construct containing an EV loading-signal containing RNA and an RNA linked thereto. The RNA construct can be used preferably in the form of a single-stranded RNA. More specifically, the RNA construct is integrated in the form of a single-stranded RNA in an expression vector (e.g., retroviral vector), and then, reverse-transcribed within a cell. This allows the RNA reverse transcript to be selectively loaded into EVs. Preferably, the RNA construct may be a construct that contains a nucleotide sequence having an identity of 90% or more to the nucleotide sequence consisting of nucleotide residues of positions 783 to 1164 in the nucleotide sequence of SEQ ID NO: 1 (wherein the nucleotide sequence corresponds to an RNA sequence having uridine residues in place of thymidine residues), as the EV loading signal containing RNA, and that is to be reverse-transcribed into RNA having an ability to be loaded into EVs.

The construct of the present invention is useful, for example, for preparing EVs containing a large amount of a functional molecule. The construct of the present invention is also useful for preparing an expression vector and a transformed cell that can be used for preparing EVs containing a large amount of a functional molecule.

### (Expression Vector)

The present invention also provides an expression vector containing a nucleic acid construct as mentioned above and a promoter operably linked thereto.

The nucleic acid construct and the promoter operably linked thereto can serve as an expression unit for the nucleic acid construct. The term "expression unit" refers to a minimum unit that contains a certain polynucleotide to be expressed as RNA or a protein and a promoter operably linked thereto and that enables transcription of the polynucleotide (production of a protein if the polynucleotide encodes the protein). The expression unit may further contain elements such as a terminator, a ribosome binding site and a gene resistant to a drug (e.g., tetracycline, ampicillin, kanamycin, hygromycin, phosphinothricin). The expression unit may be a single-stranded nucleic acid or a double-stranded nucleic acid and may be DNA or RNA.

The promoter is preferably a promoter exhibiting transcriptional activity within a cell derived from an animal such as a mammal. Examples of the promoter include viral promoters such as SV40-derived early promoter, a cytomegalovirus LTR, a Rous sarcoma virus LTR, a MoMuLV-derived LTR and adenovirus-derived early promoter; and mammal constituent protein gene promoters such as a β-actin gene promoter, a PGK gene promoter and a transferrin gene promoter. As the promoter, a promoter exhibiting tissue-or cell-specific transcriptional activity may be used. Furthermore, a non-inducible promoter or an inducible promoter can be used as the promoter. Such a promoter can be appropriately selected depending on the factor such as a host cell into which an expression vector is to be introduced.

In an embodiment, the expression vector may be a DNA vector. The DNA vector can be used preferably in the form of a double-stranded DNA. Examples of the DNA vector include plasmids, DNA viral vectors, phages and artificial chromosomes.

In another embodiment, the expression vector may be an RNA vector. The RNA vector can be used preferably in the form of single-stranded RNA. Examples of the RNA vector include RNA viral vectors (e.g., retroviral vectors) and mRNA expression particles (e.g., mRNA-encapsulated particles containing the RNA construct of the present invention in the expressible form as mRNA).

The expression vector may be an integrative vector or a non-integrative vector. The integrative vector may be a vector which is to be integrated in its entirety in a host-cell genome. Alternatively, the integrative vector may be a vector, only a part (e.g., expression unit) of which is integrated in a host-cell genome. The expression vector may further contain a region which enables homologous recombination with a host-cell genome in order to undergo efficient homologous recombination with host-cell genomic DNA. For example, the expression vector may be designed such that an expression unit contained therein is located between a pair of homologous regions (e.g., homology arms such as loxP and FRT, homologous to a certain sequence in a host-cell genome). The genomic region (target in a homology region) of the host cell into which the expression unit is to be introduced is not particularly limited, but may be a locus of a gene highly expressed in a host cell.

The expression vector of the present invention is useful, for example, for preparing EVs containing a large amount of a functional molecule. The expression vector of the present invention is also useful as a vector for use in vector introduction therapy since it can secrete EVs containing a large amount of a therapeutic nucleic acid such as an antisense nucleic acid into cells of a subject.

### (Transformed Cells)

The present invention also provides a transformed cell containing an expression unit containing a nucleic acid construct as mentioned above and a promoter operably linked thereto.

The host cell to be used for preparation of a transformed cell is preferably an animal-derived cell. Examples of the animal include mammals (e.g., primates such as humans and monkeys; rodents such as mice, rats and rabbits; ungulates such as cows, pigs, goats, horses and sheep, predatory animals such as dogs and cats) and avian species (e.g., chickens). Preferably, the animal is a mammal such as a human. More preferably, the animal is a human from the viewpoint of clinical application. The host cell may also be a primary cultured cell, a cell line derived from a primary cultured cell, a commercially available cell line or a cell line available from a cell bank. Thus, a transformed cell prepared using such a host cell is compatible with a cell derived from an animal or cell species as mentioned above.

The expression unit contained in the transformed cell is the same as the expression unit as mentioned above. The expression unit may be integrated in one or more different positions of a host-cell genomic region. The transformed cell containing such an expression unit constantly (i.e., stably) expresses an RNA transcript of a nucleic acid construct to allow selective loading of the RNA transcript into EVs. Alternatively, the expression unit may be present in a site (e.g., cytoplasm, within a nucleus) other than the host-cell genomic region. The transformed cell containing such an expression unit temporarily expresses an RNA transcript of a nucleic acid construct to allow selective loading of the RNA transcript into EVs. These transformed cells can be prepared by introducing an expression vector as mentioned above into a host cell.

In a certain embodiment, the transformed cell may be a non-cancerous cell. Accordingly, the transformed cell is not a cell prepared by transforming a cancer cell derived from a subject (e.g., human) with cancer. Examples of the non-cancerous cell include non-cancerous primary cultured cells and non-cancerous cell lines. The non-cancerous cell may be a non-stem cell or a stem cell. Examples of the stem cell include embryonic stem cells, somatic stem cells and artificial stem cells (e.g., iPS cells) derived from normal cells. The non-cancerous cell may be a cell derived from any tissue or organ. Examples of such tissues or organs include kidney, lung, digestive organs (e.g., stomach, small intestine, colon, rectum), pancreas, kidney, liver, thymus gland, spleen, thyroid, adrenal gland, prostate, bladder, ovary, uterus, bone, skin and brain.

In a preferable embodiment, the transformed cell may be a kidney cell. Examples of the kidney cell include primary cultured cells and non-cancerous cell lines (e.g., 293 cell lines such as HEK293 cell lines). The kidney cell is preferably a human kidney cell.

In a certain embodiment, the transformed cell may be a cancer cell. Examples of the cancer cell include cells derived from the tissues or organs as mentioned above. The cancer cell is preferably a breast adenocarcinoma cell (e.g., MCF7 cell line) or non-small cell lung cancer cell (e.g., NCI-H1299 cell line).

The transformed cell can be prepared by any method commonly known in the technical field. For example, the transformed cell can be prepared by a method using an expression vector as mentioned above (e.g., competent cell method, electroporation method, liposome method) or genome modification technology using a nucleic acid construct as mentioned above. If an expression vector is an integrative vector which undergoes homologous recombination with host-cell genomic DNA, an expression unit can be integrated into the host-cell genomic DNA by transformation. In contrast, if an expression vector is a non-integrative vector which does not undergo with host-cell genomic DNA, the expression unit is not integrated into the host-cell genomic DNA by transformation and can be present as it is independently of the genomic DNA within the host cell. Alternatively, an expression unit can be integrated into host-cell genomic DNA in accordance with genome editing technology (e.g., CRISPR/Cas system).

The transformed cell of the present invention is useful for preparation of EVs containing a large amount of a functional molecule. The transformed cell of the present invention is also useful as a cell for cell transplantation therapy since it can secrete EVs containing a large amount of a therapeutic nucleic acid such as an antisense nucleic acid.

### (RNA Transcript of Nucleic acid Construct)

The present invention provides an RNA transcript of a nucleic acid construct containing an EV loading signal-containing nucleic acid and a functional nucleic acid molecule transcribably linked thereto. The EV loading signal-containing nucleic acid contains an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 gene. The definitions, examples and preferable examples of the EV loading signal-containing nucleic acid, functional nucleic acid molecule, linkage and nucleic acid construct, and elements related to these are the same as mentioned above.

In a certain embodiment, an RNA transcript of a nucleic acid construct may contain an untranslated region (e.g., 5'-UTR, 3'-UTR) in the gene as mentioned above or a region constituted of an RNA transcript of a partial region of the gene having an ability to be loaded into EVs.

In another certain embodiment, an RNA transcript of a nucleic acid construct may contain a region constituted of an RNA transcript containing a nucleotide sequence having an identity of 90% or more to the nucleotide sequence consisting of nucleotide residues of positions 783 to 1164 in the nucleotide sequence of SEQ ID NO: 1 and having an ability to be loaded into EVs.

The RNA transcript of a nucleic acid construct can be extracted from the EVs obtained by a method for producing EVs as described later. Extraction can be made by destroying the membrane structure of EVs by a treatment using, e.g., a reagent (e.g., an organic solvent such as phenol, a surfactant) or physical manipulation (e.g., pressurization), or a combination thereof.

An RNA transcript of a nucleic acid construct may be purified from an extract obtained by the extraction as mentioned above. Purification can be made by any method as long as RNAs can be purified by the method. Examples of the purification method include chromatography (e.g., ion exchange chromatography, hydrophobic chromatography, reversed-phase chromatography, affinity chromatography with a nucleic acid complementary to the whole or part of an RNA transcript), methods based on molecular-weight fractionation (e.g., dialysis, ultrafiltration, gel filtration), electrophoresis (e.g., SDS polyacrylamide electrophoresis, isoelectric point electrophoresis), salting out and a precipitation method, and a combination thereof.

The RNA transcript of the present invention is useful in, e.g., the fields of reagents and medical drugs, since a functional nucleic acid molecule contained in the nucleic acid construct can be used as a component of a reagent, medical drug or the like.

### (Extracellular Vesicles)

The present invention also provides extracellular vesicles (EVs) containing an RNA transcript of a nucleic acid construct as mentioned above. The definitions, examples and preferable examples of the nucleic acid construct and RNA transcript and elements related to these are the same as mentioned above

EVs are small vesicles having a membrane structure and secreted from various types of cells. Examples of EVs include exosomes, ectosomes and apoptotic vacuoles and virus-like particles. Preferably, the EVs are exosomes. EVs can be defined by a size thereof. The size of EVs is, for example, 30 to 1000 nm, preferably 30 to 300 nm, and more preferably 30 to 200 nm. The size of EVs can be measured by NanoSight (manufactured by Malvern Instruments).

EVs can be specified by an EV marker. Examples of the EV marker include internal markers and surface markers of EVs. Examples of internal markers of EVs include carcinoembryonic antigen (CEA), CA125, CA15-3, actin family, TSG101, ALIX, Charged multivesicular body protein (CHMP) family, Glyceraldehyde 3-phosphate dehydrogenase (GAPDH), poly(ADP-ribose)polymerase (PARP) family, AFP, CA19-9, Flotillin-I, Flotillin-II, Rab protein, programmed cell death (PDCD)6, phospholipid scramblase (PLSCR) family, cytochrome C oxidase (COX) family, lamin family, proliferating cell nuclear antigen (PCNA), tubulin family, TATA binding protein (TBP), voltage-dependent anion channel protein (VDAC), amyloid beta and tau protein. Examples of surface markers of EVs include tetraspanin membrane proteins (EV membrane specific tetraspan membrane proteins, such as CD9, CD63, CD81), extracellular matrix metalloprotease induction substances (e.g., CD147), heat shock protein (HSP)70, HSP90, major tissue compatibility complex (MHC)I, lysosome-associated membrane proteins (LAMP)1, intercellular adhesion molecule (ICAM)-1, integrin, ceramide, cholesterol, phosphatidylserine, Annexins, Caveolin-I and EpCAM. The EV marker is preferably an EV surface marker, more preferably a tetraspanin membrane protein and still further preferably CD9.

EVs can be obtained by a method for producing EVs as described later.

The EVs of the present invention are useful as nucleic acid delivery vehicles in, e.g., the fields of reagents and medical drugs, since they can contain a large amount of RNA transcripts as mentioned above.

### (Method for Producing Extracellular Vesicles)

The present invention provides a method for producing extracellular vesicles (EVs).

In an embodiment, the method of the present invention may be a method for producing EVs containing a functional molecule. The method includes the following (1) and (2):
(1) introducing a construct of the present invention or an expression vector of the present invention into an animal cell; and
(2) culturing the animal cell obtained in (1) in a culture medium to secrete EVs containing a functional molecule from the animal cell into the culture medium. The definitions, examples and preferable examples of the functional molecule, EVs, construct and animal cell and elements related to these are the same as mentioned above.

A construct of the present invention or an expression vector of the present invention can be introduced into an animal cell by any method. Examples of the method include competent cell methods, electroporation methods and liposome methods.

An animal cell can be cultured under any conditions as long as the animal cell can be cultured. Examples of the culture medium for use in culturing include Minimal Essential Medium (MEM), Dulbecco's modified minimal essential medium (DMEM), F12 medium, RPMI1640 medium and a medium containing a mixed medium of them as a base medium. Examples of additives to a culture medium include various types of amino acids, various types of inorganic salts, various types of vitamins, various types of antibiotic substances and buffering agents. The culture conditions can be appropriately determined. For example, the pH of a culture medium is about 6 to about 8 and the culture temperature is usually about 30 to about 40°C. The culture medium may or may not contain serum. If the culture medium contains no serum, a serum substitute may be contained. If transplantation of a transformed cell or administration of EVs secreted from a transformed cell is intended, a serum-free medium is preferable from the viewpoint of preventing contamination with unwanted components and reducing an infection risk. If culture is performed in this way, EVs containing a functional molecule (linked to an EV loading signal-containing nucleic acid) can be secreted in a culture medium.

In another embodiment, the method of the present invention may be a method for producing EVs containing an RNA transcript. The method includes culturing a transformed cell of the present invention to produce EVs containing an RNA transcript of a nucleic acid construct, which contains an EV loading signal-containing nucleic acid and a functional nucleic acid molecule transcribably linked thereto. The definitions, examples and preferable examples of the EVs, transformed cell, EV loading signal-containing nucleic acid, functional nucleic acid molecule, linkage, nucleic acid construct and RNA transcript and elements related to these are the same as mentioned above.

A transformed cell can be cultured under any conditions as long as a host cell used for preparing the transformed cell can be cultured. The details for, e.g., a culture medium, additives and culture conditions are the same as those set forth above for culturing of animal cells. EVs containing an RNA transcript can be secreted in a culture medium by culturing as described.

The EVs secreted in a culture medium can be purified from the medium. Examples of the method for purifying EVs include centrifugation (e.g., ultracentrifugation), filtration and use of a substance having affinity with an EV membrane and a combination thereof.

In a certain embodiment, EVs may be purified from a culture medium using a substance having affinity with an EV membrane. Examples of the substance having affinity with an EV membrane include an antibody against a surface marker of EVs as mentioned above, an aptamer, a phosphatidylserine binding protein and a ceramide binding protein. In the present invention, as the substance having affinity with a surface marker of EVs, a single type of substance or a plurality of types of substances (e.g., 2, 3 and 4 types) can be used. Preferably, the substance having affinity with an EV membrane may be an antibody against a surface marker of the EV membrane from the viewpoint of ensuring specificity to the surface marker of EVs and simple preparation. Examples of the antibody include full-length antibodies (e.g., monoclonal antibodies, polyclonal antibodies) and antigen-binding fragments thereof. The antigen-binding fragment may be any antibody fragment as long as it has binding ability to a target EV surface marker. Examples of the antigen-binding fragment include Fab, Fab', F(ab')₂ and scFv.

The substance having affinity with an EV membrane may be immobilized onto a solid phase and put in use. Examples of the solid phase include beads (e.g., sepharose beads, agarose beads, magnetic beads) and supports (e.g., plate such as plastic plates, membranes). The solid phase may be magnetic beads from the viewpoint of, e.g., convenience in collection by a magnetic manipulation. Examples of the magnetic beads include beads containing a magnetic material such as iron, nickel and cobalt, or an alloy (e.g., ferrite) of these.

The method of the present invention is useful for production of an RNA transcript and EVs.

Now, the present invention will be more specifically described with reference to Examples but the present invention is not limited to the following Examples.

### Examples

### Materials and Methods

### 1. RNA-seq Analysis for HEK293 Cells

### (1) Articles

FBS (Thermo Fisher Scientific, 10437028)
MEMα medium (Fujifilm Wako Pure Chemical Industries, Ltd. 135-15175)
100x Penicillin/Streptomycin solution (Fujifilm Wako Pure Chemical Industries, Ltd. 168-23191)
CD9-bound magnetic particles (manufactured in-house)
EV binding buffer (manufactured in-house, final concentration 50 mM EDTA, 50 mM EGTA, 1% CMC, 0.5x PBS)
TRIzol (Thermo Fisher Scientific, 15596018)
NEBNext Poly(A) mRNA Magnetic Isolation Module (NEB, E7490S)
GenNext RamDA-seq Single Cell Kit (Toyobo Co., Ltd., RMD-101T)

### (2) Cell Culture

HEK293 cells (JCRB, JCRB9068) were cultured in a 10% FBS-MEMα medium (penicillin and streptomycin were added) in an ordinary manner.

The culture supernatant for use in production of EVs was prepared by culturing the cells for three days from confluent in FBS-free MEMα medium (penicillin and streptomycin were added).

### (3) EV Purification

To the culture supernatant, anti-CD9 antibody-bound magnetic particles (manufactured in-house) and an EV binding buffer (manufactured in-house) were added. They were mixed by inversion overnight at 4°C. The magnetic particles having EVs bound thereto were washed with PBS and subjected to RNA purification.

### (4) Total RNA Purification

Cells and EVs were dissolved in TRIzol and RNAs were purified in accordance with the manual.

### (5) PolyA RNA Purification

From the total RNAs purified, polyA RNAs were purified in accordance with the manual for NEBNext Poly(A) mRNA Magnetic Isolation Module.

### (6) RNA Sequencing

An RNA sequence library was prepared using the polyA RNAs in accordance with the manual for GenNext RamDA-seq Single Cell Kit, and paired-end sequence analysis (150 bp x 2) was carried out using NextSeq.

### (7) RNA Sequence Analysis

From a sequence file obtained by NextSeq analysis, gene expression levels (FPKM) were added up and tested using fastp (0.19.4), hisat2 (2.1.0) and cufflinks (1.59.0). Human release 28 (GRCh38. p12) of gencode was used as gene annotation data. Fold changes in RNA expression of cells and EVs were calculated using the calculated gene expression levels, and then, the genes having FPKM in EVs of 100 or more and significantly enriched in EVs having a enrichment rate (Log2 fold change) of 4.5 or more, were listed up.

### 2. ISO-seq Analysis for HEK293 Cells

### (1) Sample

Three samples shown in Table 1 were used in total in the analysis.

**Table 1. Samples used in this analysis**

| No. | Samples | Mediums/Materials | Volume |
|---|---|---|---|
| 1 | HEK293 cell | · MEMα (Wako, #135-15175) 500 mL | 5 mL |
| | | · FBS (Gibco, #10437-028) 57 mL | |
| | | · Penicillin-Streptomycin x 100 (Wako, #168-23191) 5.7 mL | |
| | | · MEM Non-Essential Amino Acids Solution x 100 (Wako, #139-15651) 5.7 mL | |
| 2 | EVs from HEK293 cell | Same as above | 1000 mL |
| 3 | EVs from serum | Serum | 10 mL |

### (2) Extraction of RNAs of HEK293 Cells

HEK293 cells were cultured in 5 mL of culture medium No. 1 shown in Table 1. After culture, the culture supernatant was removed and RNAs were extracted from the cells using TRIzol^{™} Reagent (Thermo fisher, #15596026) in accordance with the protocol (https://assets.thermofisher.com/TFS-Assets/LSG/manuals/trizol_J_12_Jun_2007_1.pdf).

### (3) Extraction of RNAs from HEK293 Cell-derived EVs

HEK293 cells were cultured in 1000 mL of culture medium No. 1 shown in Table 1. After culture, the culture supernatant was centrifuged by a swing rotor at 2000 G for five minutes to precipitate cells and debris. The supernatant was collected, transferred to a 0.45 µm filter (Corning, #430514) and filtered by use of a vacuum pump. The filtrate was centrifugally concentrated using an Amicon Ultra (Amicon Ultra)-15 centrifugal filter unit (Millipore, #UFC910008) at 2000 G for five minutes and the fraction trapped by the filter was recovered. After PBS was added to the filter and gently stirred by pipetting several times, PBS was collected. In this step, concentration was finally performed up to 10 mL. Thereafter, RNAs were extracted using TRIzol^{™} LS Reagent (Thermo fisher, #10296010) in accordance with the protocol (https://assets.thermofisher.com/TFS-Assets/LSG/manuals/TRIzol_LS_J1_15Nov2010.pdf).

### (4) Extraction of RNAs of Serum-derived EVs

Serum samples (1.0 mL x 10) dispensed and cryopreserved at -20°C were thawed and centrifuged at 4°C and 15,000 G for 15 minutes to precipitate cells and debris. The supernatants were collected from these and put in a single tube. Subsequently, 10 tubes containing 1.0 mL of 2 x PEVIA Ver.2 (100 mM EDTA, 100 mM EGTA, 1% CMC) were prepared and magnetic beads (10 µL) having a CD9 antibody bound thereto were added to each of the tubes. Further to each of these tubes, serum (1.0 mL) was added and mixed by a rotator (10 rpm) for 16 to 24 hours while warming at 4°C. Thereafter, 3 µL of cellulase (Sigma-Aldrich, C2605-50 ML) was added to each of the tubes and the tubes were warmed at room temperature for 10 minutes. Each tube was placed on a magnetic rack and a magnetic field was applied for five minutes, and then, the supernatant was removed. PBS (500 µL) was added to each of the tubes to suspend the magnetic beads. The suspensions of 5 mL in total were joined and separated into four tubes. A magnetic field was again applied to the tubes and the supernatant was removed. To each of the tubes, PBS (500 µL) was added to obtain a suspension. The suspensions of 2.0 mL in total were joined in a single tube. Thereafter, a magnetic field was again applied to the tube and the supernatant was removed. To the tube, TRIzol^{™} Reagent (Thermo fisher, #15596026) was added and RNAs were extracted in accordance with the protocol (https://assets.thermofisher.com/TFS-Assets/LSG/manuals/trizol_J_12_Jun_2007_1.pdf).

Samples for Iso-Seq were prepared from the samples shown in Table 1. The samples were prepared in accordance with the protocol "Procedure Checklist Iso-Seq Express Template Preparation for Sequel and Sequel II Systems.pdf" published by PacBio. The amounts of RNAs used for preparing samples were as follows: (1) HEK293 cell: 300 ng, (2) HEK293 cell-derived EVs: 300 ng, (3) serum-derived EVs: lower detection limit or less.

The amounts of DNA of each sample prepared are (1) HEK293 cell: 108 ng, (2) HEK293 cell-derived EVs: 274 ng, (3) serum-derived EVs: 13 ng.

Analysis (run) by Sequel was performed in accordance with the protocol "Procedure Checklist Iso-Seq Express Template Preparation for Sequel and Sequel II Systems.pdf" published by PacBio.

The data obtained were analyzed in accordance with the publicly available method (https://ucdavis-bioinformatics-training.github.io/2020-september-isoseq/liz/bioconda/2-bioconda). Finally, the base sequences of individual transcripts were determined.

A method for analyzing the base sequence is shown below.
(i) Converting polymerase read sequences into CCS sequences
(ii) Removing primer sequences
(iii) Removing poly A sequences and concatemers
(iv) Categorizing for every transcript
(v) Correcting by CCS leads

### 3. Analysis for Transient Expression

### (1) Articles

FBS (Thermo Fisher Scientific, 10437028)
MEMα medium (Fujifilm Wako Pure Chemical Industries, Ltd. 135-15175)
100x Penicillin/Streptomycin solution (Fujifilm Wako Pure Chemical Industries, Ltd. 168-23191)
Lipofectamin 3000 Transfection Reagent (Thermo Fisher Scientific, L300001)
CD9-bound magnetic particles (manufactured in-house)
EV binding buffer (manufactured in house, final concentration 50 mM EDTA, 50 mM EGTA, 1% CMC, 0.5x PBS)
TRIzol (Thermo Fisher Scientific, 15596018)
ReverTraAce qPCR RT Mix/gDNA Remover (Toyobo Co., Ltd., FSQ-301)
KOD SYBR qPCR Mix (Toyobo Co., Ltd., QKD-201)
Plasmids: shown below.

**Table 2. Details of plasmids**

| Name of plasmids | Sequences inserted into cloning site of pcDNA3.1 |
|---|---|
| RAB13 | RAB13 mRNA in total length (NM_002870.5) |
| ANP32B | ANP32B mRNA in total length (NM_006401.3) |
| ACTB | ACTB mRNA in total length (NM_001101.5) |

### (2) Cell Culture

HEK293 cells (JCRB, JCRB9068) were cultured in a 10% FBS-MEMα medium (penicillin and streptomycin were added) in an ordinary manner.

### (3) Transfection

Transfection was carried out in accordance with the manual attached to Lipofectamine 3000 transfection reagent. Forty eight hours after transfection, the culture medium was exchanged with a fresh complete medium. Further 24 hours later, the culture supernatant and cells were collected.

### (4) Purification of EVs

To the culture supernatant, anti-CD9 antibody-bound magnetic particles (manufactured in-house) and an EV binding buffer (manufactured in-house) were added. They were mixed by inversion overnight at 4°C. The magnetic particles having EVs bound thereto were washed with PBS and subjected to RNA purification.

### (5) Purification of Total RNAs

Cells and EVs were dissolved in TRIzol and RNAs were purified in accordance with the manual.

### (6) RT-qPCR

Using total RNAs purified, cDNAs were prepared in accordance with the manual of ReverTraAce, and then, qPCR was carried out using KOD SYBR qPCR Mix. The primer set and conditions used for quantitation are shown below.

**Table 3. A set of primers**

| Name of primers | Sequences |
|---|---|
| pcDNA3.1 univ F | ATCAGCCTCGACTGTGCCTTCTAGTT (SEQ ID NO:2) |
| pcDNA3.1 univ R | GAAAGGACAGTGGGAGTGGCACCTT (SEQ ID NO:3) |
| GAPDH F | ACTTTGTCAAGCTCATTTCCTGGTATGAC (SEQ ID NO:4) |
| GAPDH R | GGTACTTTATTGATGGTACATGACAAGGTG (SEQ ID NO:6) |

**Table 4. Conditions**

| Temperture | Time | Number of cycles |
|---|---|---|
| 98°C | 120 seconds | |
| 98°C | 10 seconds | 40 cycles |
| 60°C | 10 seconds | |
| 68°C | 30 seconds | |

### (7) Analysis

The expression levels of the transgenes obtained and the expression level of GAPDH (endogenous gene) were calculated in accordance with the following formula to obtain enrichment rates of the transgenes in EVs.
(EV-transgene/EV-GAPDH)/(Cell-transgene/Cell-GAPDH)

### 4. Analysis for Stably Expressing Cell Line (Part 1)

A deletion analysis (part 1) of RAB13 gene in Example 5 was performed as follows.

### (1) Articles

FBS (Thermo Fisher Scientific, 10437028)
MEMα medium (Fujifilm Wako Pure Chemical Industries, Ltd. 135-15175)
100x Penicillin/Streptomycin solution (Fujifilm Wako Pure Chemical Industries, Ltd. 168-23191)
Lipofectamin 3000 Transfection Reagent (Thermo Fisher Scientific, L300001)
Geneticin Selective Antibiotic (Thermo Fisher Scientific, 10131035)
CD9-bound magnetic particles (manufactured in-house)
EV binding buffer (manufactured in house, final concentration 50 mM EDTA, 50 mM EGTA, 1% CMC, 0.5x PBS)
TRIzol (Thermo Fisher Scientific, 15596018)
ReverTraAce qPCR RT Mix/gDNA Remover (Toyobo Co., Ltd., FSQ-301)
KOD SYBR qPCR Mix (Toyobo Co., Ltd., QKD-201)
Plasmids: shown below.

**Table 5. Details of plasmids**

| Name of plasmids | Sequences inserted into cloning site of pcDNA3.1 |
|---|---|
| RAB13 | RAB13 mRNA in total length (NM_002870.5) |
| ANP32B | ANP32B mRNA in total length (NM_006401.3) |
| ACTB | ACTB mRNA in total length (NM_001101.5) |
| ANP32B 5'UTR-eGFP | Plasmid in which 5'UTR sequence of ANP32B is inserted into MCS of pcDNA3.1+C-eGFP |

### (2) Cell Culture

HEK293 cells (JCRB, JCRB9068) were cultured in a 10% FBS-MEMα medium (penicillin and streptomycin were added) in an ordinary manner. G418 drug selection was carried out by adding G418 in a concentration of 250 µg/mL and exchanging the medium at an interval of one to two days for two weeks. The stably expressing cell line established was cultured in a 10% FBS-MEMα medium (penicillin and streptomycin were added) for three days to obtain a culture supernatant.

### (3) Transfection

Transfection was carried out in accordance with the manual attached to Lipofectamine 3000 transfection reagent. Forty eight hours after transfection, the culture medium was exchanged with G418 supplemented medium and subjected to drug selection for two weeks.

### (4) Purification of EVs

To the culture supernatant, anti-CD9 antibody-bound magnetic particles (manufactured in-house) and an EV binding buffer (manufactured in-house) were added. They were mixed by inversion overnight at 4°C. The magnetic particles having EVs bound thereto were washed with PBS and subjected to RNA purification.

### (5) Purification of Total RNAs

Cells and EVs were dissolved in TRIzol and RNAs were purified in accordance with the manual.

### (6) RT-qPCR

Using total RNAs purified, cDNAs were prepared in accordance with the manual of ReverTraAce, and then, qPCR was carried out using KOD SYBR qPCR Mix. The primer set and conditions used for quantitation are the same as shown in Tables 3 and 4.

### (7) Analysis

The expression levels of the transgenes obtained and the expression level of GAPDH (endogenous gene) were calculated in accordance with the following formula to obtain enrichment rates of the transgenes in EVs.
(EV-transgene/EV-GAPDH)/(Cell-transgene/Cell-GAPDH)

### 5. Analysis of Cell Line Stably Expressing RAB13 Partial Sequence

### (1) Articles

Plasmids are as shown below.

**Table 6. Details of plasmids**

| Name of plasmids | Sequences inserted into cloning site of pcDNA3.1 |
|---|---|
| RAB13 in total length | RAB13 mRNA in total length (NM_002870.5) |
| ACTB | ACTB mRNA in total length (NM_001101.5) |
| RAB13 in the former part 2/3 | RAB13 mRNA 1-802 |
| RAB13 in the later part 2/3 | RAB13 mRNA 375-1164 |
| RAB13 in the former part 1/2 | RAB13 mRNA 1-598 |
| RAB13 in the former part 1/3 | RAB13 mRNA 1-375 |
| RAB13 in the later part 1/3 | RAB13 mRNA 783-1164 |

### (2) Procedure

Cell lines stably expressing the plasmids mentioned above were prepared in the same manner as in the section of "4 Analysis for Stably Expressing Cell Line" and enrichment rates of transgenes in EVs were calculated.

### 6. Additional Experiments on Other Cell Lines

### (1) Cells

The following cells were used in place of HEK293 cells.
NCI-H1299 cells (ATCC, CRL-5803)
MCF7 cells (ATCC, HTB-22)

### (2) Procedure

Stably expressing cell lines were prepared in the same manner as in the above section "4. Analysis for Stably Expressing Cell Line" and enrichment rates of transgenes in EVs were calculated.

### 7. Experiments Using eGFP Fusion Gene

### (1) Articles

Plasmids are as shown below.

**Table 7. Details of plasmids**

| Name of plasmids | Sequences inserted into cloning site of pcDNA3.1 |
|---|---|
| RAB13 783-1164 Sense | 783-1164 of RAB13 (NM_002870.5) is cloned into pcDNA3.1(+)-N-eGFP |
| RAB13 783-1164 Antisense | Antisense to 783-1164 of RAB13 (NM_002870.5) is cloned into pcDNA3.1(+)-N-eGFP |
| RAB13 783-1164 Scramble | Scramble sequence 783-1164 of RAB13 (NM_002870.5) (shown below) is cloned into pcDNA3.1(+)-N-eGFP |

### Scramble sequence:

TGAATAAATAGAAACAAACATATCATGCAAGGGTTATTGGCCGAAATGTGATGG
AAAGAGGGTCGATTGATCAAGATGATCGTGGTAAATGGAAAAGTGACGTACACAGCTGG
AAGCTGTAAGTGATCGTAGTAGCACACAGAGCAGAGCGGGGGATGAGCATTGTAAGTGA
GCCGTGAACCTAAGGTTGAAGAAAGAGGCTTGAGTAAAAAGTAGTACAGTGAGCGGATT
AGAGAGGAGGTGAGATACGATACGTGCAGGGGGCGGAAGCTACATAGGACGTAGAGAGG
ATGCGTACAAAGGGTATTGTGCAACAGAGTCTAGGGTTAAATGAGGAATTAGCCTGTGT
ATAAGAAAGCATTGAAGTACAAAACTGTCGAGAAAGCAATGCAT (SEQ ID NO: 6)

### (2) Articles

Cell lines stably expressing the plasmids mentioned above were prepared in the same manner as in the section of "4. Analysis for Stably Expressing Cell Line" and enrichment rates of transgenes in EVs were calculated.

### 7. Analysis for stably expressing cell line (Part 2)

Deletion analysis (part 2) of RAB 13 gene in Example 8 was performed as follows. Unless otherwise specified, the same manner as in the above section "4. Analysis for stably expressing cell line (part 1)" was employed.

### (1) Articles

**Table 8. Details of plasmids**

| Name of plasmids | Sequences inserted into cloning site of pcDNA3.1 |
|---|---|
| 783_1164_S | Sense strand to RAB13 mRNA 783-1164 |
| 783_1164_AS | Antisense strand to RAB13 mRNA 783-1164 |
| 783_1037_S | Sense strand to RAB13 mRNA 783-1037 |
| 783_953_S | Sense strand to RAB13 mRNA 783-953 |
| 783_953_AS | Antisense strand to RAB13 mRNA 783-953 |
| 813_953_S | Sense strand to RAB13 mRNA 813-953 |
| 813_953_AS | Antisense strand to RAB13 mRNA 813-953 |
| 843_953_S | Sense strand to RAB13 mRNA 843-957 |
| 843_953_AS | Antisense strand to RAB13 mRNA 843-957 |
| 869-1037_S | Sense strand to RAB13 mRNA 869-1037 |

### (2) Procedure

Cell lines stably expressing the plasmids mentioned above were prepared in the same manner as in the section of "4. Analysis for Stably Expressing Cell Line (Part 1)" and enrichment rates of transgenes in EVs were calculated. At this time, only the RT-qPCR primers used in the above section 4 (6) were changed as follows.

**Table 9. Details of primers**

| Name of primers | Sequences |
|---|---|
| EGFP_Fw | GACTGGGTGCTCAGGTAGTG (SEQ ID NO:7) |
| EGFP_Rv | CAAGATCCGCCACAACATCG (SEQ ID NO:8) |
| GAPDH F | ACTTTGTCAAGCTCATTTCCTGGTATGAC (SEQ ID NO:9) |
| GAPDH R | GGTACTTTATTGATGGTACATGACAAGGTG (SEQ ID NO:10) |

### Example 1: Identification of Transcripts Selectively loaded into Extracellular Vesicles

Using HEK293 cell line, which is most frequently used as a cell line for producing therapeutic extracellular vesicles, RNAs selectively loaded into EVs were globally analyzed by transcriptome analysis for EVs and cells. The RNAs to be analyzed were all of the 60637 transcripts registered in the GENCODE database.

As a result, 8488 transcripts were found at detectable expression levels. Among these transcripts, transcripts (EV FPKM > 100 and log2 fold change > 4.5 and p < 0.001) significantly and abundantly present in EVs were narrowed down, with the result that 17 transcripts were identified (Table 8).

From these results, RAB13, LUC7L3, ANP32B, and NET1 genes, which were abundantly present in EVs, were focused and subjected to the following analysis.

**Table 10. RNA transcripts selective to extracelluar vesicles from HEK293 cells**

| gene | Cell FPKM | EV FPKM | log2 (fold_change) |
|---|---|---|---|
| RAB13 | 79.0 | 16376.6 | 7.70 |
| LUC7L3 | 62.5 | 3245.4 | 5.70 |
| ANP32B | 72.5 | 3144.3 | 5.44 |
| NET1 | 23.8 | 1097.4 | 5.53 |
| NEFM | 31.5 | 875.7 | 4.80 |
| MTURN | 20.2 | 870.2 | 5.43 |
| C1orf115 | 29.0 | 804.7 | 4.79 |
| C22orf46 | 13.4 | 632.0 | 5.56 |
| PLEKHA4 | 4.5 | 397.9 | 6.47 |
| HMGN5 | 8.4 | 359.9 | 5.43 |
| TRAK2 | 14.0 | 328.8 | 4.56 |
| RASSF3 | 10.4 | 305.6 | 4.87 |
| FBXW4 | 7.8 | 264.6 | 5.08 |
| ARRDC4 | 6.9 | 175.1 | 4.67 |
| ZSWIM9 | 2.3 | 127.9 | 5.78 |
| CA11 | 3.7 | 118.6 | 5.00 |
| LINC01952 | 0 | 30.5 | inf |

### Example 2: Analysis of Transcript Variant Selectively loaded into Extracellular Vesicles

For the purpose of analyzing whether or not transcript variants loaded into EVs among the gene products identified in Example 1 have a characteristic feature and whether or not the variants are loaded into EVs while keeping functionality, i.e., intact state, ISO-seq analysis using a long read sequencer was carried out.

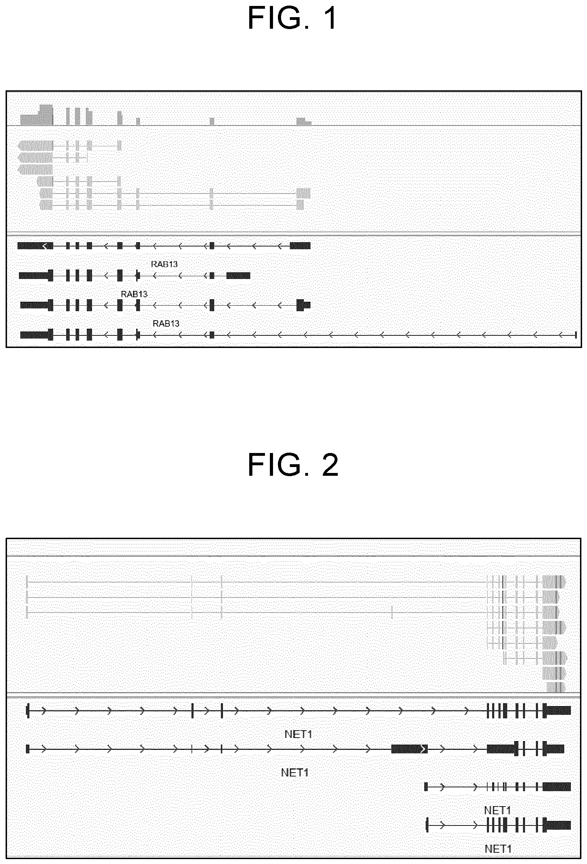

As a result, intact transcript variants of RAB13, ANP32B and NET1 genes were detected but intact transcript variants of LUC7L3 gene and other candidate genes were not confirmed (FIGs. 1 to 3). For RAB13 and NET1 genes, respectively NM_002870.5 and NM_001047160.3 among a plurality of transcript isoforms registered, were identified to be present within EVs.

### Example 3: Examination of Loading Rate to Extracellular Vesicles

Based on the results of Examples 1 and 2, the following experiments were carried out using the sequences of RAB13 and ANP32B genes with the view to establishing a "method for loading any RNA molecule into EVs". Expression vectors expressing the full-length sequences containing untranslated regions of RAB13 and ANP32B genes as well as ACTB gene were designed and transfected into HEK293 cell line, and the genes were forcibly expressed. The ACTB gene was used as a control gene that is not loaded into EVs. Each of the expression vectors was transfected, and the gene expression levels of EVs and cells were quantitatively analyzed by the RT-qPCR method. Individual expression levels were normalized by that of the endogenous gene (GAPDH), and thereafter, EV loading rates (EV/Cell) were calculated.

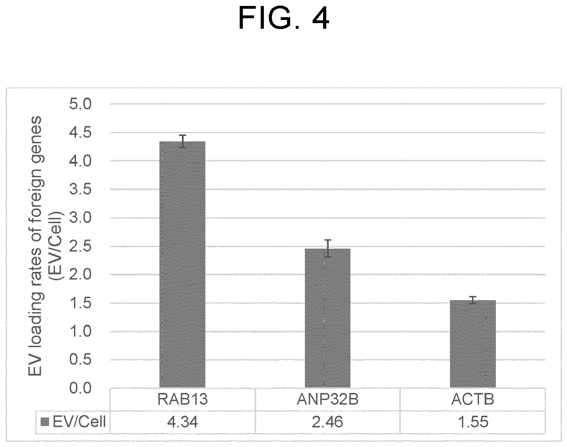

As a result, it was demonstrated that a foreign nucleic acid molecule (a region including a multi-cloning site) derived from an expression vector can be significantly loaded into EVs by use of the sequences of RAB13 and ANP32B genes (FIG. 4). The ACTB gene used as a control had an EV/Cell ratio of 2 or less. It was confirmed that the ACTB gene was not selectively loaded into EVs, as expected (FIG. 4).

### Example 4: Analysis for Function as Loading Signal to Extracellular Vesicles

In stably expressing cell lines, which were prepared by introducing foreign genes linked to the sequences of RAB13 and ANP32B genes into host-cell genomes, whether the sequences of RAB13 and ANP32B genes function as an EV loading signal, was checked. Expression vectors, which were prepared by linking a foreign gene (717 bp) encoding an enhanced green fluorescent protein (eGFP) to the 5'-untranslated regions (UTR) of individual genes contained in the expression vectors (RAB13, AN32B and ACTB gene expression vectors) as well as ANP32B gene, were introduced in the genomes of HEK293 cells to establish stably expressing cell lines. The expression levels of the foreign gene were determined and analyzed using these stably expressing cell lines in accordance with the RT-qPCR method.

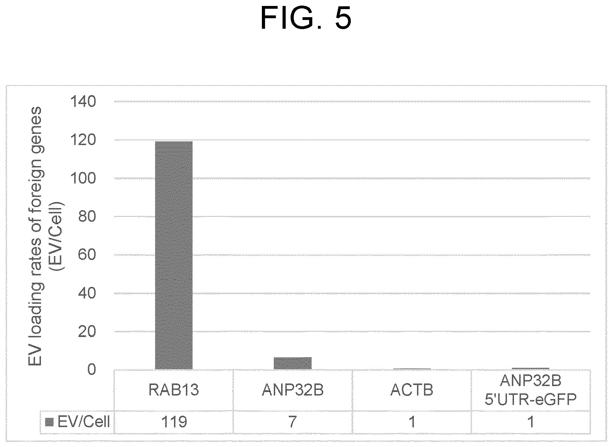

As a result, it was confirmed that the foreign genes linked to RAB13 and ANP32B genes were loaded into EVs with extremely high-efficiency (FIG. 5). In contrast, the foreign genes linked to the 5'-UTRs of ACTB and ANP32B genes exhibited an EV/Cell of 1, showing that the foreign genes were not selectively loaded (FIG. 5). In particular, it was shown that the presence of only the 5'-UTR sequence of ANP32B gene is not enough for loading into EVs (FIG. 5).

### Example 5: Deletion Analysis of RAB 13 Gene (Part 1)

For the purpose of identifying an important signal sequence for loading into EVs in the RNA sequence of RAB13 gene (NM_002870.5), expression vectors for various partial sequences were introduced into the genomes of HEK293 cells to establish stably expressing cell lines. The expression levels were measured in the same manner as described above using these stably expressing cell lines.

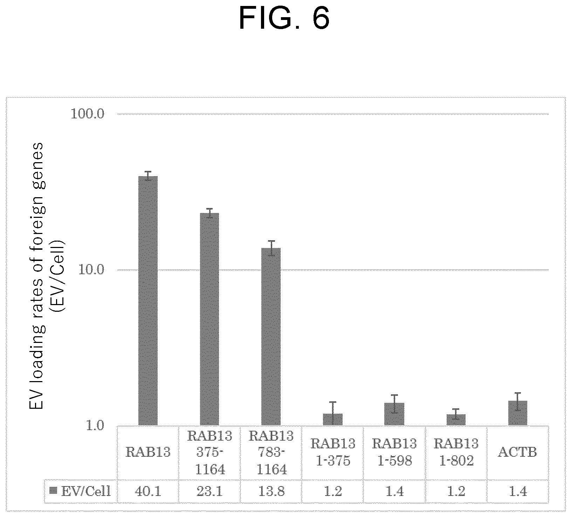

As a result, the foreign genes linked to the sequences of the full-length sequence (positions 1-1164), positions 375-1164, positions 575-1164, or positions 783-1164 of RAB13 gene represented by SEQ ID NO: 1 showed excellent loading into EVs (FIG. 6). Thus, it was suggested that the sequence containing 3'-UTR (positions 783-1164) of RAB13 gene was important as the EV loading signal. In contrast, the EV/Cell ratios of all the sequences not containing 3'-UTR (positions 783-1164) of RAB13 gene were close to 1. The result clearly showed that the sequences were not virtually enriched in EVs (FIG. 6).

### Example 6: Confirmation of Loading into Extracellular Vesicles Using Cells Other Than HEK293 Cell Line

For the purpose of confirming that the EV loading signal containing RAB13 3'-UTR (positions 783-1164) also functions in cells other than the HEK293 cell line, stably expressing cell lines were established using a lung cancer cell line (H1299 cell line) and a breast cancer cell line (MCF7 cell line) in the same manner as above. The expression levels were quantified using these stably expressing cell lines in the same manner as above.

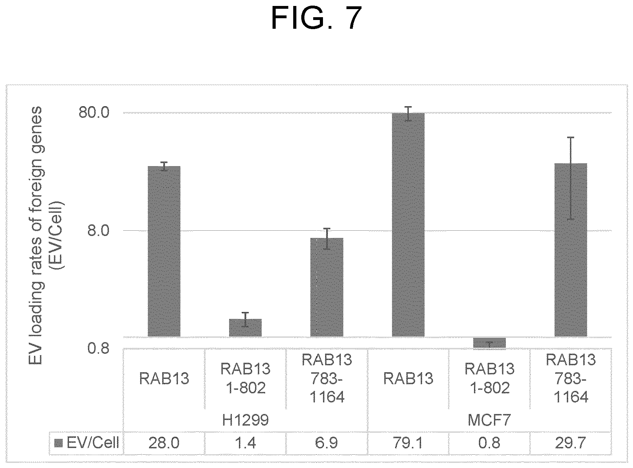

As a result, it was found that only foreign genes containing the full-length sequence of RAB13 or a sequence of positions 783-1164 selectively were loaded into EVs (FIG. 7).

### Example 7: Confirmation of Loading Using eGFP Fusion Gene into Extracellular Vesicles

For the purpose of confirming that the EV loading signal containing RAB13 3'-UTR (positions 783-1164) also functions as a fusion gene with a functional gene encoding a protein other than RAB13, an expression vector expressing a fusion gene having RAB13 3'-UTR (positions 783-1164) linked to the 3'UTR of eGFP gene was designed. Then, stably expressing cell lines were established using the HEK293 cell line in the same manner as above. The expression levels were quantified using these stably expressing cell lines in the same manner as above.

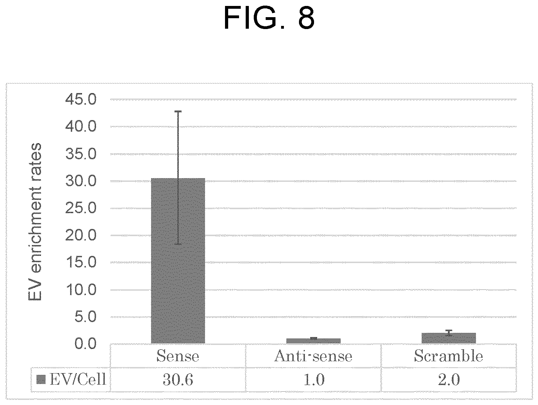

As a result, it was found that only a fusion gene prepared by linking RAB13 3'-UTR (positions 783-1164) in the forward direction was selectively s loaded into EVs (FIG. 8). Conversely, it was found that a fusion gene prepared by linking RAB13 3'-UTR (positions 783-1164) in the reverse direction and a fusion gene prepared by linking a scramble sequence of RAB13 3'-UTR (positions 783-1164) were not selectively loaded into EVs (FIG. 8).

### Example 8: Deletion Analysis of RAB 13 Gene (Part 2)

A sequence within the RAB 13 3' UTR sequence (783-1164) (serving as an EV loading signal) essential for an EV loading signal was analyzed. A stably expressing cell line expressing a gene sequence obtained by binding a whole or part of the RAB13 3'UTR sequence to the 3' terminal side of the GFP gene sequence, was established in the HEK293 cell line, and loading of the GFP gene to EVs was checked.

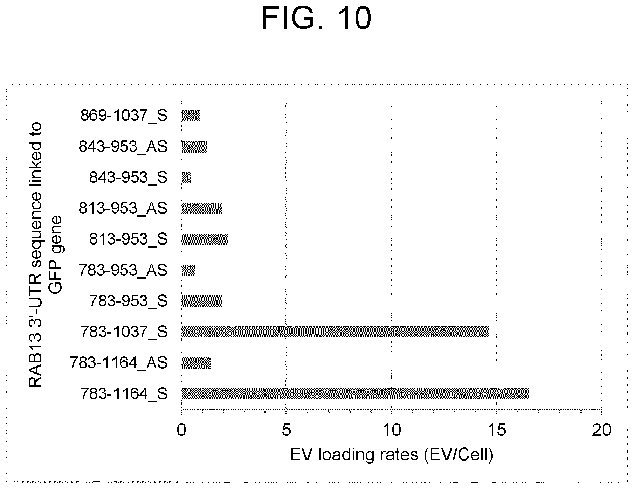

As a result, the cell line containing a 783-1037 sequence exhibited a loading rate to EVs equivalent to the cell line containing the full-length 3'UTR sequence (FIG. 10). In contrast, the cell lines containing shorter sequences than the 783-1037 sequence exhibited enrichment rates in EVs equivalent to the antisense strand serving as a comparative group and no enrichment in EVs was confirmed (FIG. 10).

Sequence Listing

## Claims

1. A construct comprising: an extracellular vesicle (EV) loading signal-containing nucleic acid; and a functional molecule linked thereto,
wherein the EV loading signal-containing nucleic acid contains an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 genes and
wherein the functional molecule is a functional molecule other than the EV loading signal-containing nucleic acid.

2. The construct according to claim 1, wherein the EV loading signal-containing nucleic acid contains an EV loading signal in one or more genes selected from the group consisting of RAB13, LUC7L3, ANP32B, NET1, MTURN, C22orf46, PLEKHA4, HMGN5, FBXW4, ZSWIM9, CA11 and LINC01952 genes.

3. The construct according to claim 1, wherein the EV loading signal-containing nucleic acid contains a 3'-untranslated region in the one or more genes or a partial region thereof having an ability to be loaded into EVs.

4. The construct according to claim 1, wherein the EV loading signal-containing nucleic acid is a nucleic acid containing (i) a nucleotide sequence consisting of nucleotide residues of positions 783 to 1164 in a nucleotide sequence of SEQ ID NO: 1 or (ii) a nucleotide sequence having an identity of 90% or more to a nucleotide sequence consisting of nucleotide residues of positions 783-1037 in a nucleotide sequence of SEQ ID NO: 1, and having an ability to be loaded into EVs.

5. The construct according to claim 1, wherein the EV loading signal-containing nucleic acid is RNA.

6. The construct according to claim 1, wherein the functional molecule is a nucleic acid molecule, an amino acid, a peptide, a protein, a lipid, a monosaccharide or a polysaccharide.

7. The construct according to claim 1, wherein the construct is a nucleic acid construct containing an EV loading signal-containing nucleic acid and a functional nucleic acid molecule linked thereto.

8. An expression vector comprising: the nucleic acid construct according to claim 7; and a promoter operably linked thereto.

9. A transformed cell comprising an expression unit containing the nucleic acid construct according to claim 7; and a promoter operably linked thereto.

10. The transformed cell according to claim 9, wherein the transformed cell is (a) a kidney cell, a breast adenocarcinoma cell or a non-small cell lung cancer cell, or (b) a HEK293 cell line, an NCI-H1299 cell line or an MCF7 cell line.

11. An RNA transcript of a nucleic acid construct comprising: an EV loading signal-containing nucleic acid; and a functional nucleic acid molecule transcribably linked thereto,
wherein the EV loading signal-containing nucleic acid contains an EV loading signal in one or more genes selected from the group consisting of RAB13, LUC7L3, ANP32B, NET1, NEFM, MTURN, C1orf115, C22orf46, PLEKHA4, HMGN5, TRAK2, RASSF3, FBXW4, ARRDC4, ZSWIM9, CA11 and LINC01952 genes, and
wherein the functional molecule is a functional nucleic acid molecule other than the EV loading signal-containing nucleic acid.

12. An EV comprising the RNA transcript according to claim 11.

13. A method for producing an EV containing a functional molecule, comprising the following (1) and (2):
(1) introducing the construct according to any one of claims 1 to 7 or the expression vector according to claim 8 into an animal cell; and
(2) culturing the animal cell obtained in (1) in a culture medium to secrete the EV containing a functional molecule from the animal cell into the culture medium.

14. The method according to claim 13, wherein the animal cell is (a) a kidney cell, a breast adenocarcinoma cell or a non-small cell lung cancer cell line, or (b) a HEK293 cell line, an NCI-H1299 cell line or an MCF7 cell line.

15. A method for producing an EV containing an RNA transcript of a nucleic acid construct containing an EV loading signal-containing nucleic acid and a functional nucleic acid molecule linked thereto, the method comprising culturing the transformed cell according to claim 9 or 10 to produce the EV containing the RNA transcript.
